# EUROPÄISCHE PATENTSCHRIFT

(11) **EP 4 008 710 B1**
(45) Veröffentlichungstag und Bekanntmachung des Hinweises auf die Patenterteilung: **01.05.2024**
(21) Anmeldenummer: 20211815.4
(22) Anmeldetag: 04.12.2020
(51) Int. Cl.: C07C 45/50, C07C 47/347

(54) **VERFAHREN ZUR HYDROFORMYLIERUNG VON OLEFINEN IN HOMOGENER PHASE**
METHOD FOR HYDROFORMYLATION OF OLEFINS IN THE HOMOGENEOUS PHASE
PROCÉDÉ D'HYDROFORMYLATION DES OLÉFINES EN PHASE HOMOGÈNE

(43) Veröffentlichungstag der Anmeldung: 08.06.2022
(73) Patentinhaber: OQ Chemicals GmbH, 40789 Monheim am Rhein (DE)
(72) Erfinder: Vogelsang, Dennis, 48249 Dülmen (DE); Hüttermann, Lars, 45899 Gelsenkirchen (DE); Wilmsen, Tim, 44629 Herne (DE)
(74) Vertreter: Michalski Hüttermann & Partner Patentanwälte mbB

(56) Entgegenhaltungen:
- EP-A1- 1 529 769
- EP-A1- 1 529 771
- DE-A1-102006 004 318

## Beschreibung

Die vorliegende Erfindung betrifft ein Verfahren zur Herstellung von Aldehyden durch Hydroformylierung von Olefinen mittels Synthesegas an einem Übergangsmetallkomplexkatalysator, wobei innerhalb eines ersten Prozessschrittes die Olefine mittels eines wasserlöslichen Übergangsmetallkomplexkatalysators aus einem Metall und daran gebundenen Liganden in Gegenwart eines mit Wasser mischbaren Lösungsmittels zur Reaktion gebracht werden, wobei der Druck, die Temperatur und die Mengenanteile des Lösungsmittels und der wässrigen Katalysatorlösung so gesteuert werden, dass die Hydroformylierung in einer homogenen einphasigen Reaktionslösung durchgeführt wird; und innerhalb eines zweiten Prozessschrittes durch Absenkung der Temperatur und/oder Reduzierung des Druckes die aus dem ersten Reaktionsschritt erhaltene homogene Reaktionslösung in eine zweiphasige Prozesslösung überführt und mindestens ein Teil der organischen Phase von der wässrigen Phase abgetrennt wird.

Die weitere Funktionalisierung von Olefinen durch Hydroformylierung mit Synthesegas in Gegenwart eines Metallkatalysators ist seit langem bekannt. Als primäre Produkte der Umsetzung werden Aldehyde mit einem Kohlenstoffatom mehr als das Edukt-Olefin erhalten. Diese Aldehyde können als solche oder bevorzugt selbst als weitere Edukte zur Herstellung einer Vielzahl nützlicher Folgeprodukte verwendet werden. Wichtige industrielle Folgeprodukte der eigentlichen Hydroformylierung sind aus den Aldehyden, beispielsweise durch Hydrierung, erhältliche Alkohole wie Butanol oder 2-Ethylhexanol, welche über die aldehydische Zwischenstufe aus Propen als Olefin-Edukt gewonnen werden können. Die letztendlichen Produkte der Hydroformylierung werden vielfältig als Lösungsmittel, als Zwischenprodukte für die Herstellung von Wasch- und Reinigungsmitteln, Schmiermittel oder Weichmacher für Kunststoffe eingesetzt.

Während die grundsätzlichen Zusammenhänge der Umsetzung der Olefine zu den Aldehyden bekannt sind, ergibt sich in großtechnischen Verfahren immer noch eine Vielzahl von Optimierungsansätzen, da die Wirtschaftlichkeit des Gesamtverfahrens durch ein komplexes Zusammenspiel aus Reaktionsbedingungen, Eduktumsatz, gewünschter Selektivität sowie Katalysatorstandzeiten und -wiedergewinnung geprägt ist. Insbesondere den Katalysatoren und deren effizientem Einsatz kommt in dieser Matrix eine herausragende Bedeutung zu, da die Kosten für die üblicherweise eingesetzten Metalle die der anderen Reaktionsteilnehmer bei weitem übersteigen.

Dies einbeziehend wurde eine Vielzahl unterschiedlicher großtechnischer Prozessmöglichkeiten entwickelt, deren unterschiedliche Verfahrensausgestaltungen sich in zahlreichen Dokumenten der Patentliteratur wiederfinden.

So offenbart beispielsweise die WO 2004 024 661 A1 ein Verfahren zur katalytischen Hydroformylierung olefinisch ungesättigter Verbindungen mit 3 bis 24 Kohlenstoffatomen, wobei als Katalysator zumindest ein Metall der 8. bis 10. Gruppe des Periodensystems der Elemente aufweisender, unmodifizierter Katalysator eingesetzt wird, wobei die Hydroformylierung in Gegenwart eines cyclischen Kohlensäureesters der folgenden Formel 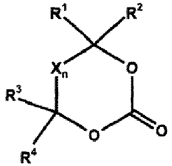
- R1, R², R³, R⁴:: jeweils gleich oder verschieden: H, substituierte oder unsubstituierte, aliphatische, alicyclische, aromatische, aliphatisch-alicyclische, aliphatisch-aromatische, alicyclisch-aromatische Kohlenwasserstoffreste mit 1 bis 27 C-Atomen.
- n:: 0 - 5
- X:: zweiwertiger substituierter oder unsubstituierter, aliphatischer, alicyclischer, aromatischer, aliphatisch-alicyclischer, aliphatisch-aromatischer Kohlenwasserstoffrest mit 1 bis 27 C-Atomen.
durchgeführt wird, wobei der Anteil des Kohlensäureesters zumindest 1 Gew.-% des Reaktionsgemisches beträgt.

In weiteren Patentdokumenten, EP 1 529 771 A1 und EP 1 529 769 A1, werden Verfahren zur Herstellung von 8(9)-Formyl-tricyclo[5.2.1.0^{2.6}]dec-3-en durch Hydroformylierung von Dicyclopentadien in einem heterogenen Reaktionssystem unter Verwendung einer wässrigen Lösung offenbart, wobei wasserlösliche organische Phosphor(III)-Verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas umgesetzt werden, wobei man als wasserlösliche organische Phosphor(III)-Verbindungen spezielle sulfonierte Triarylphosphine benutzt.

Eine weitere Möglichkeit zur Umsetzung zyklischer Verbindungen mit mehreren Doppelbindungen wird in der DE 10 2006 004 318 A1 offenbart. Diese beschreibt ein Verfahren zur Herstellung von 3(4),7(8)-Dihydroxymethyl-bicyclo[4.3.0]nonan durch Hydroformylierung von Bicyclo[4.3.0]nona-3,7-dien mit nachfolgender Hydrierung, wobei man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bisformyl-bicyclo[4.3.0]nonan anschließend zum 3(4),7(8)-Dihydroxymethyl-bicyclo[4.3.0]nonan hydriert.

Derartige aus dem Stand der Technik bekannte Lösungen können noch weiteres Verbesserungspotential bieten, insbesondere hinsichtlich der möglichen Effizienz der Umsetzung, der Einfachheit der Auftrennung des Reaktionsgemisches sowie der Möglichkeit der Wiederverwendbarkeit des eingesetzten Katalysatormaterials.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein Verfahren bereitzustellen, welches eine effiziente Umsetzung auch schwierig zu hydroformylierender Olefine und des Weiteren eine einfache und effiziente Auftrennung der Reaktionslösung mit verbesserter Wiederverwendung des Katalysators ermöglicht.

Die Lösung der Aufgabe erfolgt durch die Merkmale des unabhängigen Verfahrensanspruchs. Bevorzugte Ausgestaltungen der Erfindung sind in den Unteransprüchen, in der Beschreibung oder den Figuren angegeben, wobei weitere in den Unteransprüchen oder in der Beschreibung oder den Figuren beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Erfindungsgemäß gelöst wird die Aufgabe durch ein Verfahren zur Herstellung von Aldehyden durch Hydroformylierung von Olefinen mittels Synthesegas an einem Übergangsmetallkomplexkatalysator, wobei innerhalb eines ersten Prozessschrittes die Olefine mittels eines wasserlöslichen Übergangsmetallkomplexkatalysators aus einem Metall und daran gebundenen Liganden in Gegenwart eines mit Wasser mischbaren Lösungsmittels zur Reaktion gebracht werden, wobei der Druck, die Temperatur und die Mengenanteile des Lösungsmittels und der wässrigen Katalysatorlösung so gesteuert werden, dass die Hydroformylierung in einer homogenen einphasigen Reaktionslösung durchgeführt wird; und innerhalb eines zweiten Prozessschrittes durch Absenkung der Temperatur und/oder Reduzierung des Druckes die aus dem ersten Reaktionsschritt erhaltene homogene Reaktionslösung in eine zweiphasige Prozesslösung überführt und mindestens ein Teil der organischen Phase von der wässrigen Phase abgetrennt wird.

Überraschenderweise wurde gefunden, dass über das erfindungsgemäße Verfahren in der gesteuert einphasigen Reaktionsumgebung mit wässriger Katalysatorkomponente sich eine Vielzahl unterschiedlicher Olefine hoch selektiv zu den entsprechenden Aldehyden umsetzten lassen, wobei auch bei Einsatz schwer umzusetzender Olefin-Edukte, mit ein oder mehreren Doppelbindungen und/oder ein oder mehreren Ringstrukturen, hohe Umsätze innerhalb kurzer Prozesszeiten erreicht werden. Neben der sehr effizienten Umsetzung innerhalb der Reaktionsumgebung wird durch die gesteuerte Zusammensetzung der Reaktionsumgebung zudem auch die Aufarbeitung der Reaktionslösung nach Reaktionsende in einem erheblichen Maß erleichtert. Hier ergibt sich als weiterer Vorteil, dass eine einfache und thermisch schonende Abtrennung der gewünschten organischen Produkte und der Katalysatorlösung möglich ist, welches eine einfache Aufarbeitung und den Wiedereinsatz des kostenintensiven Katalysators ohne nennenswerte Reaktivitäts- und Stoffverluste ermöglicht. Ohne durch die Theorie gebunden zu sein, scheint die einphasige Reaktionsumgebung unter den gegebenen Reaktionsbedingungen den Katalysator, oder speziell auch die organischen Liganden des Katalysatorkomplexes, vor einem frühzeitigen Abbau zu schützen. Des Weiteren scheint die gesteuerte Überführung der Reaktionslösung in ein zweiphasiges System aus organischer Produkt- und wässriger Katalysatorphase im zweiten Verfahrensschritt einen signifikanten Verlust an Katalysatorsystem in der Aufarbeitung zu verhindern, wobei sowohl die rückgewinnbare Menge wie auch die Aktivität des Katalysators im Vergleich zu anderen Katalysator-Aufarbeitungsverfahren höher ist. Dies gilt insbesondere im Vergleich zu Umsetzungen in rein organischer Phase, welche Schwierigkeiten bei der Rückgewinnung des nicht wasserlöslichen Katalysators zeigen. Die wasserlöslichen Katalysatoren werden nach der Umsetzung durch mechanische Separierung der wässrigen Katalysatorphase von der organischen Produktphase getrennt. Damit wird das wasserlösliche Katalysatorsystem, im Gegensatz zu den destillativen Auftrennungen bei Umsetzungen in rein organischer Phase, thermisch nicht belastet. Eine hohe und andauernde thermische Belastung des Katalysators führt oft zu seiner Deaktivierung.

Insofern ergibt sich durch die erfindungsgemäße Kopplung der beiden Verfahrensschritte ein synergistischer Effekt, welcher hoch effiziente und katalysatorschonende Umsetzungen im großtechnischen Umfeld fördert.

Das erfindungsgemäße Verfahren ist ein Verfahren zur Herstellung von Aldehyden durch Hydroformylierung von Olefinen mittels Synthesegas an einem Übergangsmetallkomplexkatalysator. Als Edukte können Olefine oder Olefin-Gemische mit 4 bis 24 Kohlenstoffatomen, vorzugsweise mit 6 bis 20 Kohlenstoffatomen und besonders bevorzugt mit 8 bis 20 Kohlenstoffatomen eingesetzt werden. Die Gemische können Olefine mit einer oder mehreren end- und/oder innenständigen C-C-Doppelbindungen aufweisen. Die Gemische können Olefine mit gleicher, ähnlicher (± 2) oder deutlich unterschiedlicher (>±2) Kohlenstoffatomzahl (C-Zahl) aufweisen oder aus diesen bestehen. Es kann sich um geradkettige, verzweigte oder cyclische Olefine handeln. Die Olefine können aliphatische Olefine mit keiner, einer oder mehreren Ringstrukturen sein. So können die Olefine ein, zwei, drei oder mehr Ringe im Molekül aufweisen, wobei die Doppelbindungen in oder an den Ringstrukturen vorliegen können. Üblicherweise werden diese Olefine in herkömmlichen Hydroformylierungsverfahren nur unvollständig umgesetzt. Aus diesen Olefinen wird durch die Reaktion mit Synthesegas, d.h. Wasserstoff und Kohlenmonoxid, ein Aldehyd (HC=O) erhalten, wobei die C-Anzahl des Olefins durch die Reaktion um 1 erhöht wird.

In der Hydroformylierungsstufe des ersten Verfahrensschrittes wird vorzugsweise ein Synthesegas aus Kohlenmonoxid und Wasserstoff eingesetzt, bei dem das molare Verhältnis von Kohlenmonoxid zu Wasserstoff bevorzugt von 1:4 bis 4:1, besonders bevorzugt von 1:2 bis 2:1, ganz besonders bevorzugt von 1:1,2 bis 1,2:1 beträgt. Insbesondere kann ein Synthesegas eingesetzt werden, bei dem ein in etwa stöchiometrisches Verhältnis von Kohlenmonoxid und Wasserstoff vorliegt.

Innerhalb des ersten Prozessschrittes werden die Olefine mittels eines wasserlöslichen Übergangsmetallkomplexkatalysators aus einem Metall und daran gebundenen Liganden umgesetzt. Einsetzbare Hydroformylierungskatalysatoren umfassen ein Metall und ein oder mehrere an das Metall koordinierte Liganden. Die Katalysatoren können als solche präformiert in die Reaktionsumgebung gegeben werden oder aber der aktive Katalysator bildet sich unter den Reaktionsbedingungen in situ aus einer anderen Metallquelle, wie beispielsweise einem Metallsalz, in der Reaktionszone. Letzteres beispielsweise durch Anlagerung oder Austausch von in der Reaktionszone vorliegenden Liganden wie beispielsweise CO, Wasserstoff oder organischen Komplexliganden. Bevorzugt kann es sich bei dem Übergangsmetallkomplexkatalysator um ein Metall der 8.-10. Nebengruppe des Periodensystems und insbesondere um Co, Ru, Rh, Pd, Pt, Os oder Ir, speziell um Rh, Co, Ir oder Ru handeln. Die Wasserlöslichkeit des Katalysatorkomplexes wird dabei im Wesentlichen über die Wahl der Liganden mitbestimmt, wobei der Katalysator erfindungsgemäß dann wasserlöslich ist, wenn die Löslichkeit des Katalysators bei 20°C in Wasser größer oder gleich 100 g/L beträgt. Die Löslichkeit kann beispielsweise nach einem OECD-Verfahren ("OECD Guideline For The Testing Of Chemicals", "Water solubility" vom 27.07.1995) bestimmt werden. Die Liganden können anorganische Gegenionen wie beispielsweise Halogene oder komplexere organische Moleküle wie Acetate oder aromatische Komplexliganden mit einem oder mehreren Heteroatomen sein.

Die Edukte werden in der ersten Verfahrensstufe in Gegenwart eines mit Wasser mischbaren Lösungsmittels zur Reaktion gebracht. Die Hydroformylierung des Olefins über Zutritt von Synthesegas zu den Aldehyden erfolgt in der Gegenwart eines weiteren Lösemittels, welches nicht Wasser ist. Als Lösemittel können sich bevorzugt Flüssigkeiten anbieten, welche mit Wasser zumindest eine gewisse Löslichkeit aufweisen. Diese Löslichkeit des Lösemittels in Wasser kann bei 20°C beispielweise größer oder gleich 20 g/L betragen. Geeignete Lösemittel in dieser Gruppe können beispielsweise niedere Monoalkohole oder Diole sein. Das Lösemittel kann auch bevorzugt vollständig mit Wasser mischbar sein. Der Einsatz von Lösemitteln in diesem Löslichkeitsverhältnis zu Wasser kann dazu beitragen, dass besonders robuste einphasige Gebiete erhalten werden, welche auch möglicherweise auftretende Druck und/oder Temperaturschwankungen sicher ausgleichen können. Zudem kann der Einsatz einer solchen Gruppe an Lösemittel dazu beitragen, dass große Änderungen in der Zusammensetzung durch die Bildung von Produkten kompensiert werden können. Des Weiteren kann der Anteil der Lösemittel hinreichend klein gehalten werden, welches die Auftrennung des entstehenden Gemisches erleichtert und Energiekosten zur Abtrennung geringhält.

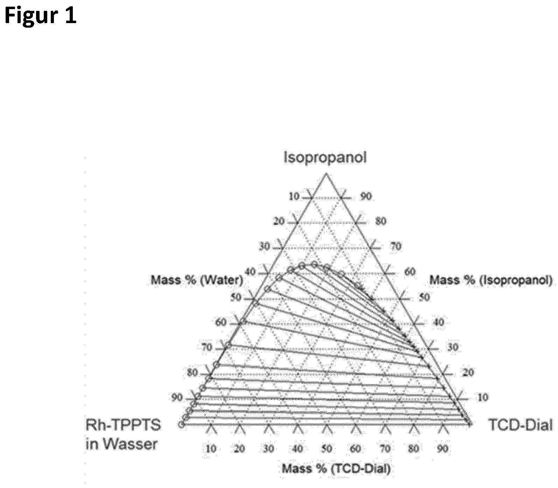

Der Druck, die Temperatur und die Mengenanteile des Lösungsmittels und der wässrigen Katalysatorlösung werden im ersten Verfahrensschritt so gesteuert, dass die Hydroformylierung in einer homogenen einphasigen Reaktionslösung durchgeführt wird. Durch das Vorliegen einer wässrigen Katalysatorlösung, nicht wasserlöslicher olefinischer Edukte sowie nur gering wasserlöslicher aldehydischer Produkte, bildet sich ohne weitere Maßnahmen in der Reaktionszone unter den Reaktionsbedingungen eine zweiphasige Lösung aus, welche einer effizienten Umsetzung der Edukte entgegensteht. Durch den Zusatz des weiteren Lösemittels können im Phasendiagramm, in Abhängigkeit der Menge wässriger Katalysator- Menge Lösemittel - Menge Edukt oder Menge Produkt/Zwischenprodukt, unter der Reaktionsbedingungen entweder ein- oder zweiphasige Bereiche vorliegen. Der Wechsel zwischen dem ein- oder zweiphasigen Bereich lässt sich über den Einsatz der Mengen insbesondere der wässrigen Katalysatormenge und des Lösemittels so steuern, dass die Reaktion unter den gegebenen Druck und Temperaturbedingungen immer im einphasigen Bereich abläuft. Durch das richtige Mengenverhältnis zwischen wässriger Katalysatorlösung und Lösemittel, zusammen mit der richtigen Lösbarkeit oder Mischbarkeit des Lösemittels mit Wasser, lässt sich die Reaktion, auch bei wechselnden oder wachsenden Anteilen an Zwischen- sowie Endprodukten, im einphasigen Bereich fahren. Die Steuerung kann beispielsweise dadurch erfolgen, dass zu Beginn der Reaktion das nötige Mengenverhältnis aus Lösemittel und wässriger Katalysatorlösung vorgegeben wird. Es ist aber auch möglich im Verlaufe der Reaktion die Mengen der einen oder anderen Komponente anzupassen, sodass im Phasendiagramm immer im einphasigen Gebiet gefahren wird. Letztere Fahrweise kann beispielsweis durch eine gesteuerte Zugabe von Lösemittel in die Reaktionszone erfolgen, welches beispielsweise auf die Menge der gebildeten Produkte angepasst ist. Die prinzipiellen ein- oder zweiphasigen Phasenbereiche des Phasendiagramms lassen sich in der Literatur finden, berechnen (seihe Figur 1) oder mit überschaubarem Aufwand durch orientierende Versuche mit wechselnden Zusammensetzungen aus gewähltem Katalysator in wässriger Lösung, Lösemittel sowie Edukt/Produkt unter Reaktionsbedingungen bestimmen.

Innerhalb eines zweiten Prozessschrittes wird durch Absenkung der Temperatur und/oder Reduzierung des Druckes die aus dem ersten Reaktionsschritt erhaltene homogene Reaktionslösung in eine zweiphasige Prozesslösung überführt und mindestens ein Teil der organischen Phase von der wässrigen Phase abgetrennt. Aus der möglichen Menge an einphasigen Zusammensetzungen aus wässriger Katalysatorlösung, Lösemittel und olefinischem Edukt/Produkt, unter den gewählten Reaktionsbedingungen, lassen sich mit einfachen orientierenden Versuchen die Untermenge möglicher Zusammensetzungen bestimmen, welche durch die Änderung der Temperatur und/oder des Drucks zu einen Wechsel im Phasenbereich führen. Diese Zusammensetzungen sind dann geeignet und in der Lage, nach Ende der Umsetzung über eine einfache Änderung der Reaktionsbedingungen in die Zweiphasigkeit zu fallen und auf diese Art und Weise eine einfache Separierung zwischen organischer und wässriger Phase herbeizuführen. Dieser Teilbereich des einphasigen Gebiets liegt in der Regel näher an der Phasengrenzlinie zwischen ein- und zweiphasigem Bereich und die Phasentrennung als Funktion der Druck- und/oder Temperaturbedingungen kann beispielsweise rein optisch verfolgt werden. Die Abtrennung der beiden Phasen kann beispielsweise über rein mechanische Schritte, wie Dekantieren erfolgen. Es ist aber auch möglich, dass die beiden Phasen ohne mechanische, rein über thermische Trennmethoden voneinander separiert werden. Durch die Zweiphasigkeit der Reaktionslösung können auch thermische Trennoperationen einfacher und ressourcenschonender durchgeführt werden. Natürlich ist es zur Aufarbeitung auch möglich, mechanische mit thermischen Trennoperationen zu koppeln, wobei die thermische Belastung des Katalysatorsystems durch den niedrigen Siedepunkt des gewählten Lösemittels in dem erfindungsgemäßen Verfahren gering ist.

In einer bevorzugten Ausgestaltung des Verfahrens kann die Temperatur und der Druck während der Umsetzung konstant gehalten und die Einphasigkeit der Reaktionslösung über die Massenverhältnisse von Lösungsmittel zur wässrigen Katalysatorlösung eingestellt werden. Es hat sich für eine effiziente Verfahrensführung zudem als vorteilhaft herausgestellt, dass der gewünschte einphasige Phasenbereich während der Reaktion im Wesentlichen über die Massenverhältnisse zwischen wässriger Katalysatorlösung und Lösemittel eingestellt wird. Es können hier "sichere" Phasenbereiche eingestellt werden, so dass die Veränderung der Zusammensetzung im Laufe der Reaktion durch die Bildung der Zwischen- und/oder Endprodukte und auch die Bildung höhermolekularer Nebenprodukte sicher kompensiert werden können. Zudem kann über die Wahl der geeigneten Lösemittel und deren Menge im Verhältnis zur wässrigen Katalysatorlösung auch eventuell auftretende Temperatur- und/oder Druckschwankungen während der Reaktion kompensiert werden. Über diese Festlegung lässt sich die Reaktion in ihrer Gesamtheit sicher im einphasigen Bereich halten. Weiter bevorzugt kann diese Steuerung über die Wahl der Mengen an Lösemittel und wässriger Katalysatorlösung zu Beginn der Reaktion festgelegt werden.

In einer bevorzugten Ausführungsform des Verfahrens kann der erste Prozessschritt bei einem Druck von größer oder gleich 0,5 MPa und kleiner oder gleich 10 MPa und bei einer Temperatur von größer oder gleich 70°C und kleiner oder gleich 150°C durchgeführt werden. Aufgrund der gesteigerten Reaktivität mit dem Synthesegas durch Fahren im einphasigen Bereich, hat sich oben angegebenes Temperaturintervall als besonders vorteilhaft herausgestellt. Es werden in diesem Bereich besonders schnelle und selektive Umsetzungen erhalten, wobei insbesondere auch der Anteil an gebildeten Hochsiedern sehr niedrig gehalten werden kann. Zudem ergeben sich deutlich verlängerte Katalysatorstandzeiten, welches wahrscheinlich auf einen verringerten Abbau der Liganden in der Reaktionslösung zurückgeführt werden kann. Überraschend hat sich zudem gezeigt, dass innerhalb dieses Temperaturbereiches besonders niedrige Drücke auch zur Umsetzung schwieriger Edukte ausreichen. Dies gilt insbesondere auch für sterisch herausfordernde Edukte oder doppelte Umsetzungen bei Einsatz von Diolefin-Edukten. Auch in diesen Umsetzungen scheint die wässrige Komponente im einphasigen Gebiet nicht den Zutritt des Synthesegases zu hemmen, sodass insgesamt schon geringere Drücke für einen ausreichenden Gaseintrag in die Reaktionslösung sorgen können.

Innerhalb eines weiter bevorzugten Aspektes des Verfahrens können die Olefine mindestens zwei nicht konjugierte Doppelbindungen aufweisen. Das erfindungsgemäße Verfahren eignet sich insbesondere für die Umsetzung herausfordernder Edukte, welche beispielsweise zwei oder mehr isolierte Doppelbindungen aufweisen können. Es hat sich gezeigt, dass die Umsetzung auch zweier Doppelbindungen innerhalb einer einphasigen Reaktion möglich ist. Dies ist erstaunlich, da im zweiphasigen Bereich durchgeführte Reaktionen in der Regel nur die Umsetzung einer Doppelbindung erlauben. Zudem kann im Vergleich zu zweiphasigen Reaktionen die Umsetzung einphasig schneller, selektiver und unter Bildung eines geringeren Anteils an hochsiedenden Nebenprodukten erfolgen. Die Doppelbindungen der Diene können innerhalb aliphatischer Ketten und/oder Zyklen vorliegen. Bevorzugt können kurz- oder mittelkettige aliphatische Di- oder höhere Olefine umgesetzt werden. So ist es möglich, Olefine mit zwei oder mehreren ungesättigten Bindungen mit einem Molekulargewicht von größer oder gleich 50 g/mol und kleiner oder gleich 500 g/mol umzusetzen.

Im Rahmen einer bevorzugten Charakteristik des Verfahrens können die Olefine mindestens einen aliphatischen Ring aufweisen. Es hat sich zudem als besonders vorteilhaft herausgestellt, dass innerhalb der erfindungsgemäßen einphasigen Verfahrensführung auch sterisch schwierig umzusetzende Olefin-Edukte umgesetzt werden können, welche ein rigides aliphatisches Ringgerüst aufweisen. Üblicherweise reagieren diese Olefine im Vergleich zu kurzen aliphatischen Ketten in Lösung mit den Katalysatoren deutlich schlechter oder bleiben im Falle mehrerer Doppelbindungen auf der Stufe eines Zwischenproduktes stehen. Die Edukte können mono- oder polyzyklisch sein. Einphasige Umsetzungen in rein organischen Lösemitteln und in rein organischer Phase sind zwar möglich, führen aber zu erhöhten Problemen in der Aufreinigung der erhaltenen Produkte. Des Weiteren zeigen letztere Umsetzungen Probleme in der Katalysatorrückgewinnung sowie dessen Standzeiten. So z.B. werden bei Umsetzungen in rein organischen Lösungsmitteln auch unmodifizierte (ohne Zugabe von Liganden) Metall-Katalysatoren für die Umsetzung der hier genannten Diolefine eingesetzt, für welche hohe Katalysatormengen benötigt werden und der Katalysator nicht rezykliert wird. Bei Einsatz von Ligand-Metall-Komplexen als Katalysatoren werden für die Hydroformylierung der polyzyklischen Diolefinen oft nicht die gewünschten Aktivitäten erreicht. Die mono- bi- oder tri-zyklischen Olefine umfassen zwei oder drei geschlossene, nicht aromatische Ringe und können des Weiteren bevorzugt ein Molekulargewicht von größer oder gleich 60 g/mol und kleiner oder gleich 450 g/mol aufweisen.

In einer weiter bevorzugten Ausgestaltung des Verfahrens kann das molare Verhältnis von Wasser in der eingesetzten wässrigen Katalysatorlösung zu Katalysatormetall, ausgedrückt als mol Wasser dividiert durch mol Katalysatormetall, größer oder gleich 5000 und kleiner oder gleich 60000 betragen. Trotz der Tatsache, dass der Zutritt der organischen Edukte an den Katalysator durch ein eher organisches Umfeld verbessert werden sollte, hat sich oben angegebenes Verhältnis von Wasser und Katalysator als besonders günstig herausgestellt. Mit diesen Wasseranteilen werden auch für Dien-Edukte vollständige Umsetzungen zu den Dialdehyden erreicht und die Verfahrensführung kann auch sicher einphasig ausgestaltet werden. Zusätzlich können Schwankungen der Reaktionsbedingungen sicher ausgeglichen werden, ohne dass das einphasige Phasengebiet verlassen wird. Dieses Wasser-Katalysatormetall-Verhältnis scheint zudem auch dazu geeignet, eine sichere und vollständige Abtrennung der wässrigen Katalysatorphase im zweiten Verfahrensschritt zu ermöglichen. Durch diese Wasseranteile wird der Katalysator und dessen Liganden sowohl in der einphasigen Umsetzung hinreichend geschützt. Zudem kann auch nach Wechsel der Reaktionsbedingungen und Zerfall der Ein- in eine Zweiphasigkeit auch eine hinreichende wässrige Umgebung aufrechterhalten werden, welches die Wiederverwendbarkeit der Katalysatorlösung fördert.

Nach einer weiter bevorzugten Ausgestaltung des Verfahrens kann das Metall des wasserlöslichen Übergangsmetallkomplexkatalysators Rhodium sein und die Liganden wasserlösliche Diphosphine oder Triarylphosphine umfassen. Der eingesetzte Katalysator oder das sich in der Reaktionslösung bildende Katalysatorsystem umfasst das Übergangsmetall Rhodium. Dieses Metall kann in einphasigen Lösungen besonders schnelle Reaktionskinetiken erlauben und kann auch sterisch schwierige Polyene oder Olefine mit starren Ringgerüsten sicher umsetzen. Neben dem Metall weist der Katalysator zumindest einen Diphosphin-Liganden mit zwei Phosphoratomen oder einen oder mehrere organische Triarylphosphin-Liganden mit einem Phosphoratom in seiner Koordinationssphäre auf oder koordiniert diese in der Reaktionslösung unter den Reaktionsbedingungen. Das katalytisch aktive System wird unter den Reaktionsbedingungen zudem durch weiteren Zutritt von Wasserstoff und Kohlenmonoxid in der Reaktionslösung gebildet, wobei die Komponenten des Synthesegases mit dem Metall einen koordinativen Komplex ausbilden. Es ist aber auch möglich, den Katalysator zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Die Triarylphosphine können beispielsweise folgender allgemeinen Formel 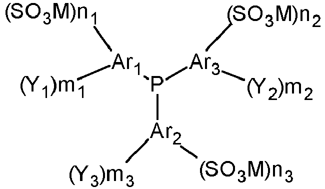 entsprechen. In dieser Formel stehen Ar₁, Ar₂ und Ar₃ für gleiche oder verschiedene Arylgruppen mit 6 bis 14 Kohlenstoffatomen. Die Substituenten Y₁, Y₂ und Y₃ stehen für gleiche oder verschiedene geradkettige oder verzweigte Alkyl- oder Alkoxyreste mit 1 bis 4 Kohlenstoffatomen, Chlor, Brom, Hydroxyl-, Cyanid- oder Nitrogruppen, sowie ferner für Aminogruppen der Formel NR¹R², wobei die Substituenten R¹ und R² gleich oder verschieden sein können und für Wasserstoff, geradkettige oder verzweigte Alkylgruppen mit 1 bis 4 Kohlenstoffatomen stehen. Die Gegenkationen M können für Lithium, Natrium, Kalium, Magnesium, Calcium oder Barium stehen, wobei m1, m2 und m3 gleich oder verschieden sein können und ganze Zahlen von 0 bis 5 bedeuten, in der n1, n2 und n3 gleich oder verschieden sind und für ganze Zahlen von 0 bis 3 stehen, wobei mindestens eine der Zahlen n1, n2 und n3 gleich oder größer 1 ist. Ein Triarylphosphin-Komplexkatalysator ist dann ein wasserlöslicher Triarylphosphin-Komplexkatalysator, wenn die Löslichkeit des Komplexes in Wasser bei 20°C größer oder gleich 100 g/L beträgt.

Zu den wasserlöslichen Triarylphosphinen der oben angegebenen Formel zählen bevorzugt solche Triarylphosphine, bei denen die Gruppen Ar₁, Ar₂, Ar₃ Phenylgruppen sind; Y₁, Y₂ und Y₃ für eine Methyl-, eine Ethylgruppe, für eine Methoxy-, Ethoxygruppe und/oder für ein Chloratom stehen. Die kationischen Reste M der anorganischen Kationen können bevorzugt Natrium, Kalium, Calcium und Barium sein. Insbesondere können solche wasserlöslichen Triarylphosphine geeignet sein, bei denen Ar₁, Ar₂, Ar₃ jeweils für eine Phenylgruppe stehen; m1, m2, m3 gleich 0 sind, n1, n2 und n3 gleich 0 oder 1 sind und n1+n2+n3 zusammen 1 bis 3 ausmachen, wobei die Sulfonat-Gruppen bevorzugt in meta-Stellung stehen können. Die Liganden können als solche oder als Mischungen eingesetzt werden. Geeignete Beispiele für wasserlösliche Triarylphosphin-Liganden sind (Sulfophenyl)-diphenylphosphin, Di-(sulfophenyl)phenylphosphin und Tri(sulfophenylphosphin). Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt. Diese Liganden können zu einer hinreichenden Wasserlöslichkeit des Katalysatorkomplexes beitragen und sind sowohl im ein- wie auch im zweiphasigen Bereich stabil.

Als wasserlösliche Diphosphine eignen sich ebenfalls sulfonierte Diphosphine der allgemeinen Formeln (III) und (IV)

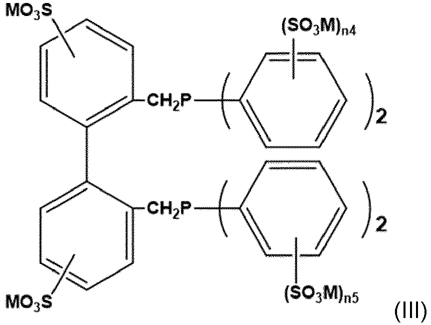

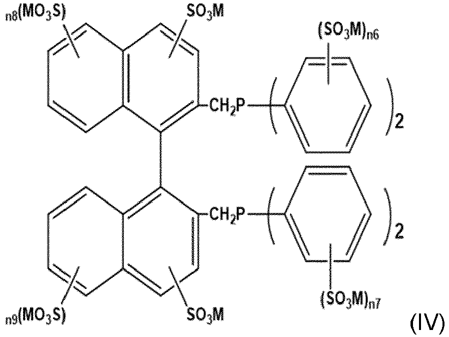

In (III) steht ein jedes n4 und n5 unabhängig voneinander für 0 oder 1, wobei die Verbindung der Formel (III) bis sechs -SOsM-Gruppen enthalten kann.

In (IV) steht ein jedes n6, n7, n8 und n9 unabhängig voneinander für 0 oder 1, wobei die Verbindung der Formel (IV) vier bis acht -SOsM-Gruppen enthält.

In den Formeln (III) und (IV) steht M für Ammonium, ein einwertiges Metall oder das Äquivalent eines mehrwertigen Metalls, insbesondere für Natrium, Kalium, Calcium oder Barium.

In einer bevorzugten Ausführungsform des Verfahrens kann das Massenverhältnis von wässriger Katalysatorlösung zu Lösungsmittel, ausgedrückt als Masse Katalysatorlösung dividiert durch Masse Lösungsmittel, größer oder gleich 0,25 und kleiner oder gleich 4 betragen. In der einphasigen Reaktionslösung hat sich oben angegebenes Massenverhältnis als besonders sicher und vorteilhaft herausgestellt. Über dieses Massenverhältnis lassen sich auch verfahrensbedingte, unvermeidliche Schwankungen des Drucks und der Temperatur, sowie die Änderung der Zusammensetzung durch die Bildung der Produkte sicher kompensieren. Weiterhin vorteilhaft kann sich eine, bezogen auf die Masse der Katalysatorlösung, relativ geringe Menge an Lösemittelzusatz von größer oder gleich 0,5 und kleiner oder gleich 2, insbesondere bevorzugt von größer oder gleich 0,75 und kleiner oder gleich 1,5, ergeben. Dieser geringe Lösemittelzusatz kann zu einer effizienteren Aufarbeitung der Reaktionslösung nach Reaktionsende beitragen.

In einem weiter bevorzugten Aspekt des Verfahrens kann das mit Wasser mischbare Lösungsmittel bei 20°C eine Löslichkeit in Wasser von größer oder gleich 20 g/L aufweisen. Um einen möglichst stabilen einphasigen Bereich mit möglichst wenig Lösungsmittelzusatz zu erhalten, hat es sich also besonders geeignet herausgestellt, dass das Lösungsmittel eine Mischbarkeit mit Wasser innerhalb des oben angegeben Bereiches aufweist. Derartige einphasige Reaktionslösungen können gegenüber den Änderungen der Reaktionsumgebung durch die hinzutretenden Produkte und auch gegenüber möglichen Änderungen der Reaktionsparameter Druck und Temperatur stabil sein und zudem noch eine besonders günstige Phasentrennung der organischen und wässrigen Phase nach Reaktionsende bewirken. Insbesondere kann der Anteil wiedergewinnbaren Katalysators nach Reaktionsende durch diese Gruppe an Lösungsmitteln erhöht sein. In einer weiter bevorzugten Ausgestaltung kann die Löslichkeit des Lösungsmittels größer oder gleich 60 g/l, des Weiteren bevorzugt größer oder gleich 70 g/l und des Weiteren bevorzugt größer oder gleich 80 g/l bei 20°C betragen. In einer weiter bevorzugten Ausgestaltung kann das Lösungsmittel mit Wasser bei 20°C vollständig mischbar sein.

In einer bevorzugten Charakteristik des Verfahrens kann das Lösungsmittel aus der Gruppe bestehend aus geradkettigen oder verzweigten C2-C5 Alkoholen oder Mischungen mindestens zweier Alkohole aus dieser Gruppe ausgesucht sein. Insbesondere die kurzkettigen Alkohole haben sich zum Erhalt besonders effizienter Umsetzungen in einem einphasigen Gebiet als besonders geeignet herausgestellt. Mittels dieser Lösemittel lassen sich auch schwierig zu hydroformylierende Edukte innerhalb sehr kurzer Prozesszeiten an wasserlöslichen Katalysatoren hoch selektiv umsetzten. Auch können die Standzeiten der Katalysatoren durch diese Lösemittelwahl deutlich verlängert werden. Diese Lösemittel zeigen im Vergleich zum Liganden eine geringe Bindungsaffinität zum Metall. Zugleich können sie sich aber stabilisierend auf den Liganden und damit vor Abbau schützend auswirken. Ein weiterer Vorteil ergibt sich dadurch, dass mit nur einem geringen Massenanteil an Lösemittel sehr robuste einphasige Gebiete erreicht werden können, welches die Kosten der Aufarbeitung und Abtrennung der gewünschten Produkte reduziert. Zudem kann die Lösemittelauswahl dazu beitragen, dass die Änderung des Systems von ein- auf zweiphasig im zweiten Verfahrensschritt sehr schnell und vollständig vonstattengeht, sodass ein Großteil des Katalysators zurückgewonnen und gegebenenfalls wieder in den Reaktionskreislauf zurückgeführt werden kann. Des Weiteren sind die niedrigen Siedepunkte der ausgewählten Lösemittel vorteilhaft, wodurch sie bei Bedarf leicht vom Produkt oder vom Katalysatorsystem abgetrennt werden können.

In einem weiter bevorzugten Aspekt des Verfahrens kann das Lösungsmittel Isopropanol sein. Die Verwendung von Isopropanol zur einphasigen Umsetzung von Olefinen im Rahmen einer Hydroformylierung hat sich als besonders günstig herausgestellt. Es lassen sich durch Isopropanolzusatz auch schwierig zu hydroformylierende Olefin-Edukte innerhalb sehr kurzer Prozesszeiten an wasserlöslichen Katalysatoren hoch selektiv umsetzten. Auch können die Standzeiten der Katalysatoren durch dieses Lösemittel deutlich verlängert werden. Ein weiterer Vorteil ergibt sich dadurch, dass mit nur einem geringen Isopropanolanteil sehr robuste einphasige Gebiete ausgebildet werden, welches die Kosten der Aufarbeitung und Abtrennung der gewünschten Produkte reduziert. Zudem kann durch die physikalischen Unterschiede des Isopropanols zu den Aldehyd-Produkten eine besonders einfache und vollständige Trennung nach Reaktionsende erreicht werden. Dies kann insbesondere auch die Wiederverwertbarkeit der Katalysatoren verlängern.

Innerhalb einer weiter bevorzugten Ausgestaltung des Verfahrens kann mindestens ein Ligand des wasserlöslichen Übergangsmetallkomplexkatalysators ein Triphenylphosphin-3,3',3"-trisulfonsäure Natriumsalz umfassen. Der Einsatz dieser Triphenylphosphin-Liganden hat sich für das Arbeiten im einphasigen Bereich als besonders vorteilhaft herausgestellt. Neben einer hochselektiven Umsetzung der eingesetzten Olefine werden, auch bei langen Reaktionszeiträumen, besonders geringe Mengen an Hochsiedern gebildet. Dies ergibt sich insbesondere bei dem Einsatz von Isopropanol als Lösungsmittel, wobei in diesen Fällen sich ein besonders geringer Abbau des organischen Liganden einstellt. Zudem können Katalysatorkomplexe mit diesen Liganden besonders effizient aus dem Reaktionsgemisch wiederverwertet werden, etwa durch Rückführung in den Reaktionskreislauf in einer kontinuierlichen Reaktion. Zudem scheint der Abbau dieser Liganden in der einphasigen Reaktionsumgebung besonders gering zu sein. In einer weiter bevorzugten Ausführungsform kann das Triphenylphosphin-3,3',3"-trisulfonsäure Natriumsalz der einzige aromatische Komplexligand der Umsetzung sein.

In einer weiter bevorzugten Ausgestaltung des Verfahrens kann der wasserlösliche Übergangsmetallkomplex-katalysator Triarylphosphin-Liganden und ein Katalysatormetall umfassen, wobei das molare Einsatzverhältnis von Triarylphosphin-Liganden zu Katalysatormetall, ausgedrückt als mol Triarylphosphin-Liganden dividiert durch mol Katalysatormetall, größer oder gleich 3 und kleiner oder gleich 15 beträgt. Für die Umsetzungen im einphasigen Bereich hat es sich als vorteilhaft herausgestellt, dass das Verhältnis von organischen Liganden zu Katalysatormetall in einem engen Bereich gehalten wird. Innerhalb dieser Verhältnisse ergeben sich sehr reproduzierbare Umsetzungen mit hohen Selektivitäten. Dies ist wahrscheinlich deshalb möglich, da eine geringere Liganden-Konzentration höhere Aktivitäten des Katalysators erlaubt. Zugleich kann der Abbau des organischen Liganden in der einphasigen Lösung verzögert oder sogar ganz verhindert werden. Somit kann diese Verfahrensführung dazu beitragen, dass der Katalysator häufiger und länger verwendet werden kann. Dieses Verhältnis trägt zudem verstärkt dazu bei, dass der Katalysator auch während der Aufarbeitung geschützt wird, sodass eine verbesserte Rückgewinnbarkeit des Katalysators nach der Abtrennung der Reaktionsprodukte resultiert. Weiter bevorzugt kann das Verhältnis größer oder gleich 5 und kleiner oder gleich 12 und insbesondere bevorzugt größer oder gleich 7 und kleiner oder gleich 10 betragen.

In einer weiter bevorzugten Ausgestaltung des Verfahrens kann das molare Verhältnis von Katalysatormetall zu Olefin, ausgedrückt als mol Katalysatormetall dividiert durch mol Olefin, größer oder gleich 0,05 % und kleiner oder gleich 0,75 % betragen. Durch die einphasige Reaktionsführung kann auch sehr effizient mit einem besonders geringen Verhältnis von Katalysator zu Olefin-Edukt gefahren werden. Es werden innerhalb kurzer Reaktionszeiten vollständige Umsetzungen zu den Dialdehyden erreicht, wobei auch die KatalysatorStandzeiten im Vergleich zu den Stand-der-Technik-Lösungen länger sein können. Das Verhältnis kann des Weiteren bevorzugt größer oder gleich 0,15 % und kleiner oder gleich 0,65 %, insbesondere bevorzugt größer oder gleich 0,3 % und kleiner oder gleich 0,5% betragen.

Innerhalb einer bevorzugten Ausführungsform des Verfahrens kann im zweiten Prozessschritt durch Absenkung der Temperatur die aus dem ersten Reaktionsschritt erhaltene homogene Reaktionslösung in eine zweiphasige Prozesslösung überführt und das Produktaldehyd abgetrennt werden. Im zweiten Prozessschritt des erfindungsgemäßen Verfahrens, nach der Umsetzung des Olefins zum Aldehyd, kann prinzipiell der Reaktionsdruck und/oder die Reaktionstemperatur reduziert werden. Damit wird die Trennung der im ersten Reaktionsschritt erhaltenen homogenen Reaktionslösung in ein zweiphasiges System erzwungen. Die eine Phase enthält den wässrigen Katalysator, wohingegen die zweite Phase das Produktaldehyd enthält. Insbesondere die Trennung der beiden Phasen hauptsächlich über die Temperatur erlaubt ein schonendes mechanisches Separieren des Produktes vom Katalysator. Dazu wird der Druck erst dann signifikant geändert, nachdem die Temperatur um mindestens 50°C von der Temperatur der Reaktionszone geändert wurde. Eine signifikante Änderung des Reaktionsdruckes ergibt sich bei einer Abweichung von mindestens 10% bezogen auf den Reaktionsdruck. Besonders vorteilhaft ist, dass keine rein thermische Abtrennung in Form einer Destillation vollzogen wird, bei welcher Höhersieder bevorzugt gebildet und das Katalysatorsystem deaktiviert werden kann. Es handelt sich um eine Prozessmethode, die unter milden Bedingungen durchgeführt wird.

Innerhalb einer weiteren Ausführungsform des Verfahrens kann der pH-Wert der wässrigen Katalysatorlösung größer oder gleich pH 4 und kleiner oder gleich pH 10 betragen. Es hat sich herausgestellt, dass durch Einstellen des pH-Wertes in dem bevorzugten Bereich der Katalysator aus dem Übergangsmetall und dem wasserlöslichen Organophosphorliganden eine sehr hohe Aktivität und hohe Selektivität bezüglich der Produktbildung aufweist. Die Einstellung kann durch bekannte Stellmittel wie anorganische Säuren oder Basen an der eingesetzten Katalysatorlösung erfolgen. Es ist aber auch möglich und vorteilhaft, dass die mittels der eingesetzten wässrigen Katalysatorlösung sich ausbildende homogene Phase auf den beschriebenen pH-Wertebereich gehalten wird. Des Weiteren wurde bei der bevorzugten pH-Wert-Einstellung niedrige Zersetzung des Katalysators beobachtet. In einer weiter bevorzugten Ausführungsform kann der pH-Wert zwischen größer oder gleich pH 5 und kleiner oder gleich pH 8, des Weiteren bevorzugt zwischen größer oder gleich pH 5,5 und kleiner oder gleich pH 7 eingestellt werden.

In einer bevorzugten Ausführungsform des Verfahrens kann das eingesetzte Olefin ein polyzyklisch aliphatisches Diolefin aus der Gruppe bestehend aus bi- oder tri-zyklischen Dienen oder Mischungen daraus ausgesucht sein. Es hat sich als besonders vorteilhaft herausgestellt, dass innerhalb der erfindungsgemäßen Verfahrensführung sterisch schwierig umzusetzende zyklische Dien-Edukte mit innenständigen Doppelbindungen umgesetzt werden können, welche durch ihr rigides Ringgerüst in Lösung mit den Katalysatorkomplexen deutlich schlechter reagieren als beispielsweise kurze aliphatische Ketten. Die polyzyklischen, olefinischen Aliphaten lassen sich durch übliche Verfahrensführungen in zweiphasigen Gebieten nur sehr unvollständig umsetzen. Einphasige Umsetzungen in rein organischen Lösemitteln sind zwar möglich, führen aber zu erhöhten Problemen in der Aufreinigung der erhaltenen Produkte. Des Weiteren zeigen letztere Umsetzungen Probleme in der Katalysatorrückgewinnung sowie dessen Standzeiten. So z.B. werden bei Umsetzungen in rein organischen Lösungsmitteln auch unmodifizierte (ohne Zugabe von Liganden) Metall-Katalysatoren für die Umsetzung der hier genannten Diolefine eingesetzt, für welche ein hoher Katalysatoreinsatz benötigt und der Katalysator nicht rezykliert wird. Unter Einsatz von Liganden-Metall-Komplexen als Katalysatoren werden für die Hydroformylierung der polyzyklischen Diolefine oft nicht die gewünschten Aktivitäten erreicht. Die erfindungsgemäß umsetzbaren bi- oder tri-zyklischen Diene umfassen zwei oder drei geschlossene, nicht aromatische Ringe und können des Weiteren bevorzugt ein Molekulargewicht von größer oder gleich 60 g/mol und kleiner oder gleich 450 g/mol aufweisen.

In einer weiter bevorzugten Ausführungsform des Verfahrens kann das Olefin ein zyklisches aliphatisches Diolefin aus der Gruppe bestehend aus Dicyclopentadien oder Norbornadien ausgesucht sein. Über die erfindungsgemäße Umsetzung lassen sich insbesondere sterisch schwierig umzusetzende sowie schlecht wasserlösliche polyzyklische aliphatische Olefine wie das Tricyclo[5.2.1.02,6]deca-3,8-dien und das Bicyclo[2.2.1]hepta-2,5-dien besonders effizient umsetzen. Es werden hohe Umsätze bei hohen Selektivitäten erzielt, wobei das Katalysatorsystem auch eine besonders lange Standzeit und eine verbesserte Rückgewinnbarkeit aufweisen kann.

### Beispiele

In einer erfindungsgemäßen Hydroformylierung wird Dicyclopentadien DCDP mittels eines organisch modifizierten Rhodium-Komplexkatalysators in einer homogenen Reaktionslösung zu dem entsprechenden Dialdehyd nach folgender Reaktionsgleichung: 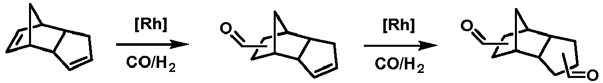 umgesetzt. Als Katalysator wird ein wasserlöslicher Komplexkatalysator eingesetzt, welcher phosphororganische TPPTS-Liganden nach folgender Formel umfasst:

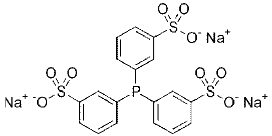

Als Lösemittel zur Erreichung der Einphasigkeit der Reaktionssystems wird Isopropanol verwendet. Die Umsetzung erfolgt bei 130°C und einem Druck von 5 MPa in einem gerührten Reaktorkessel (800 U/min) innerhalb einer Reaktionszeit von 3 h.

Die Einsatzmengen der Edukte ergeben sich wie folgt:

| **Bestandteil** | **Einsatzmenge** |
|---|---|
| Gesamt-Rhodium Konzentration in wässriger Katalysatorlösung | 46380 mg/l |
| Menge wässrige Katalysatorlösung | 2,32 ml |
| Rhodium in mol | 1,04387 mmol |
| Ligand in g | 18,38 g |
| Ligand in mol | 568 mmol |
| P/Rh-Verhältnis | 10:1 |
| Olefin in % bezogen auf gesamten Einsatz | 10% |
| Olefin in g | 50,0 g |
| Olefin in mol | 0,378 mol |
| Rhodiummenge bezogen auf Olefin | 0,46% |
| Einsatzmenge Isopropanol | 135 g |
| Einsatzmenge Wasser | 135 g |
| Synthesegaszusammensetzung H₂:CO | 1:1 |

Es wurde eine wässrige Katalysatorlösung aus Rhodium und dem Liganden hergestellt. Als Rhodiumquelle wurde Rh(OAc)₂ eingesetzt. Diese Lösung wurde mit der oben angegebenen Menge Isopropanol in den Reaktor gegeben. Dicyclopentadien wurde zudosiert und bei 5 MPa Synthesegas Druck und 130 °C für 3 h zur Reaktion gebracht. Nach Abkühlen und Entspannen des Autoklavs wurde das Isopropanol vom Reaktionsgemisch bei 100 mbar und 40°C entfernt. Nachfolgend wurde der Rückstand in einen Phasentrenner gegeben, wodurch die Separation der Katalysator- und Produktphase ermöglicht wurde. Die Produktphase wurde mittels GC untersucht. Es ergab sich folgende Zusammensetzung (ermittelt mittels GC):

| **Bestandteil (ohne Lösemittel)** | **Flächen-%** |
|---|---|
| Vorlauf | 0,48 |
| DCP | 1,50 |
| TCD-Monoenal-Isomere | 3,38 |
| TCD-Dial-Isomere | 89,92 |
| TCD-OH | 2,20 |
| Nachlauf | 2,52 |

Die Ergebnisse ergeben sich ohne Berücksichtigung des Lösemittelanteils. Die Ergebnisse zeigen, dass mit einer sehr geringen Katalysatorkonzentration von 0,45 mol-% bezogen auf das Diolefin ein fast 100%iger Umsatz erreicht wurde. Zudem ergab sich eine TCD Dial-Selektivität von ca. 90%.

Es wurde eine wiederholte Umsetzung von DCPD zu TCD unter Zusatz von Isopropanol in einem einphasigen Bereich der Reaktionslösung durchgeführt. Die Versuchsbedingungen waren jeweils wie folgt:
- 30 g DCPD pro Lauf
- 350 ppm Rh(OAc)₂ bezogen auf 300g Gesamtmasse
- P/Rh-Verhältnis= 10/1, entspricht 18,38 g TPPTS in 135 g Wasser
- 135 g Isopropanol
- Reaktionstemperatur: 130°C
- Reaktionsdruck: 50 bar
- Reaktionszeit: 3 h

Die Umsätze und Selektivitäten der einzelnen Umsetzungen entsprachen im Rahmen der Fehlergenauigkeit den oben angegebenen Ergebnissen. Der Reaktionslösung wurde nach Reaktionsende bei 100 mbar und 40 °C das Isopropanol über einen Rotationsverdampfer entzogen und der Rückstand ausgewogen. Danach wurde der restliche Rückstand in einen Phasentrenner überführt und bei RT die Phasentrennung vollzogen. Die organische Phase bestehend aus Reaktionsprodukten wurde abgenommen, ausgewogen und auf Rhodiumgehalt und Produktanteile beprobt. Die obere wässrige Katalysatorphase wurde ebenfalls ausgelassen, ausgewogen und mit dem zuvor entfernten Isopropanol wieder vereinigt. Zu der vereinigten Katalysatorphase wurde frisches DCPD zugegeben und das Gemisch erneut im Reaktor unter 130 °C und 50 bar Druck Synthesegas für 3 h zur Reaktion gebracht. Dieser Vorgang wurde iterativ fünfmal wiederholt.

Die einzelnen Auswaagen der organischen Phase über die 5 Läufe und deren Zusammensetzung insbesondere bezüglich TCD-Dial und TCD-Monoenal sind konstant. Ebenso konstant und niedrig ist der Anteil an Rhodium in der organischen Phase. Der Anteil des Rhodiums in der organischen Phase beträgt pro Lauf ca. 3,2 ppm +- 0,8 ppm% bezogen auf den gesamten Rhodiumeinsatz. Somit konnte gezeigt werden, dass neben einer erfolgreichen Umsetzung auch schwierig zu hydroformylierender polyzyklischer Diolefine mit dem erfindungsgemäßen Verfahren auch eine sehr effiziente und einfache Wiedergewinnung und -Einsatz des Katalysators möglich ist.

Die Figur 1 zeigt das Ergebnis des berechneten Phasenverhaltens eines Drei-Komponenten Gemisches als Funktion der Zusammensetzung bei einer Temperatur von 120°C. Als Komponenten liegen die wässrige Lösung eines Rh-TPPTS-Komplexkatalysators (unten links), Isopropanol als Lösungsmittel (oben) und das Endprodukt TCD-Dial (unten rechts) vor. Der gestrichelte Bereich umfasst die Massenanteile der ternären Zusammensetzungen, welche bei dieser Temperatur zwei-phasig vorliegen. Im nicht-gestrichelten, oberen Bereich des Dreiecks liegen Zusammensetzungen vor, welche unter den Druck- und Temperaturbedingungen einphasig sind. Da sich die Löslichkeiten der Olefin-Edukte nicht signifikant von den Löslichkeiten der Zwischen- und Endprodukte unterscheiden, ist dieses Phasendiagramm für die gesamte Umsetzung mit wechselnden Edukt-/Produkt-Verhältnissen repräsentativ.

## Patentansprüche

1. Verfahren zur Herstellung von Aldehyden durch Hydroformylierung von Olefinen mittels Synthesegas an einem Übergangsmetallkomplexkatalysator, **dadurch gekennzeichnet, dass** innerhalb eines
- ersten Prozessschrittes die Olefine mittels eines wasserlöslichen Übergangsmetallkomplexkatalysators aus einem Metall und daran gebundenen Liganden in Gegenwart eines mit Wasser mischbaren Lösungsmittels zur Reaktion gebracht werden, wobei der Druck, die Temperatur und die Mengenanteile des Lösungsmittels und der wässrigen Katalysatorlösung so gesteuert werden, dass die Hydroformylierung in einer homogenen einphasigen Reaktionslösung durchgeführt wird; und innerhalb eines
- zweiten Prozessschrittes durch Absenkung der Temperatur und/oder Reduzierung des Druckes die aus dem ersten Reaktionsschritt erhaltene homogene Reaktionslösung in eine zweiphasige Prozesslösung überführt und mindestens ein Teil der organischen Phase von der wässrigen Phase abgetrennt wird.

2. Verfahren nach Anspruch 1, wobei die Temperatur und der Druck während der Umsetzung konstant gehalten werden und die Einphasigkeit der Reaktionslösung über die Massenverhältnisse von Lösungsmittel zur wässrigen Katalysatorlösung eingestellt werden.

3. Verfahren nach einem der vorhergehenden Ansprüche, wobei der erste Prozessschritt bei einem Druck von größer oder gleich 0,5 MPa und kleiner oder gleich 10 MPa und bei einer Temperatur von größer oder gleich 70°C und kleiner oder gleich 150°C durchgeführt wird.

4. Verfahren nach einem der vorhergehenden Ansprüche, wobei die Olefine mindestens zwei nicht konjugierte Doppelbindungen aufweisen.

5. Verfahren nach einem der vorhergehenden Ansprüche, wobei die Olefine mindestens einen aliphatischen Ring aufweisen.

6. Verfahren nach einem der vorhergehenden Ansprüche, wobei das molare Verhältnis von Wasser in der eingesetzten wässrigen Katalysatorlösung zu Katalysatormetall, ausgedrückt als mol Wasser dividiert durch mol Katalysatormetall, größer oder gleich 5000 und kleiner oder gleich 60000 beträgt.

7. Verfahren nach einem der vorhergehenden Ansprüche, wobei das Metall des wasserlöslichen Übergangsmetallkomplexkatalysators Rhodium ist und die Liganden wasserlösliche Diphosphine oder Triarylphosphine umfassen.

8. Verfahren nach einem der vorhergehenden Ansprüche, wobei das Massenverhältnis von wässriger Katalysatorlösung zu Lösungsmittel, ausgedrückt als Masse Katalysatorlösung dividiert durch Masse Lösungsmittel, größer oder gleich 0,25 und kleiner oder gleich 4 beträgt.

9. Verfahren nach einem der vorhergehenden Ansprüche, wobei das mit Wasser mischbare Lösungsmittel bei 20°C eine Löslichkeit in Wasser von größer oder gleich 20 g/L aufweist.

10. Verfahren nach einem der vorhergehenden Ansprüche, wobei das Lösungsmittel ausgesucht ist aus der Gruppe bestehend aus geradkettigen oder verzweigten C2-C5 Alkoholen oder Mischungen mindestens zweier Alkohole aus dieser Gruppe.

11. Verfahren nach einem der vorhergehenden Ansprüche, wobei das Lösungsmittel Isopropanol ist.

12. Verfahren nach einem der vorhergehenden Ansprüche, wobei mindestens ein Ligand des wasserlöslichen Übergangsmetallkomplexkatalysators ein Triphenylphosphin-3,3',3"-trisulfonsäure Natriumsalz umfasst.

13. Verfahren nach einem der vorhergehenden Ansprüche, wobei der wasserlösliche Übergangsmetallkomplexkatalysator Triarylphosphin-Liganden und ein Katalysatormetall umfasst, wobei das molare Einsatzverhältnis von Triarylphosphin-Liganden zu Katalysatormetall, ausgedrückt als mol Triarylphosphin-Liganden dividiert durch mol Katalysatormetall, größer oder gleich 3 und kleiner oder gleich 15 beträgt.

14. Verfahren nach einem der vorhergehenden Ansprüche, wobei das molare Verhältnis von Katalysatormetall zu Olefin, ausgedrückt als mol Katalysatormetall dividiert durch mol Olefin, größer oder gleich 0,05 % und kleiner oder gleich 0,75 % beträgt.

15. Verfahren nach einem der vorhergehenden Ansprüche, wobei der pH-Wert der wässrigen Katalysatorlösung größer oder gleich pH 4 und kleiner oder gleich pH 10 beträgt.

## Claims

1. Process for the preparation of aldehydes by hydroformylation of olefins by means of synthesis gas over a transition metal complex catalyst, **characterized in that** within a
- first process step the olefins are reacted by means of a water-soluble transition metal complex catalyst comprising a metal and ligands bonded thereto in the presence of a water-miscible solvent, the pressure, the temperature and the proportions of the solvent and the aqueous catalyst solution being controlled in such a way that the hydroformylation is carried out in a homogeneous single-phase reaction solution and within a
- second process step, by lowering the temperature and/or reducing the pressure, the homogeneous reaction solution obtained from the first reaction step is converted into a two-phase process solution and at least a part of the organic phase is separated from the aqueous phase.

2. The process according to claim 1, wherein the temperature and pressure are kept constant during the reaction and the single phase of the reaction solution is adjusted via the mass ratios of solvent to aqueous catalyst solution.

3. The process according to any one of the preceding claims, wherein the first process step is performed at a pressure of greater than or equal to 0.5 MPa and less than or equal to 10 MPa and at a temperature of greater than or equal to 70°C and less than or equal to 150°C.

4. The process according to any one of the preceding claims, wherein the olefins have at least two non-conjugated double bonds.

5. The process according to any one of the preceding claims, wherein the olefins comprise at least one aliphatic ring.

6. The process according to any one of the preceding claims, wherein the molar ratio of water in the used aqueous catalyst solution to catalyst metal, expressed as moles of water divided by moles of catalyst metal, is greater than or equal to 5000 and less than or equal to 60000.

7. The process according to any one of the preceding claims, wherein the metal of the water-soluble transition metal complex catalyst is rhodium and the ligands comprise water-soluble diphosphines or triarylphosphines.

8. The process according to any one of the preceding claims, wherein the mass ratio of aqueous catalyst solution to solvent, expressed as mass of catalyst solution divided by mass of solvent, is greater than or equal to 0.25 and less than or equal to 4.

9. The process according to any one of the preceding claims, wherein the water-miscible solvent has a solubility in water at 20°C of greater than or equal to 20 g/L.

10. The process according to any one of the preceding claims, wherein the solvent is selected from the group consisting of straight or branched C2-C5 alcohols or mixtures of at least two alcohols from this group.

11. The process according to any one of the preceding claims, wherein the solvent is isopropanol.

12. The process according to any one of the preceding claims, wherein at least one ligand of the water-soluble transition metal complex catalyst comprises a triphenylphosphine-3,3',3"-trisulfonic acid sodium salt.

13. The process according to any one of the preceding claims, wherein the water-soluble transition metal complex catalyst comprises triarylphosphine ligands and a catalyst metal, wherein the molar ratio of triarylphosphine ligands to catalyst metal, expressed as moles of triarylphosphine ligands divided by moles of catalyst metal, is greater than or equal to 3 and less than or equal to 15.

14. The process according to any one of the preceding claims, wherein the molar ratio of catalyst metal to olefin, expressed as moles of catalyst metal divided by moles of olefin, is greater than or equal to 0.05% and less than or equal to 0.75%.

15. The process according to any one of the preceding claims, wherein the pH of the aqueous catalyst solution is greater than or equal to pH 4 and less than or equal to pH 10.

## Revendications

1. Procédé de fabrication d'aldéhydes par hydroformylation d'oléfines au moyen d'un gaz de synthèse sur un catalyseur complexe de métal de transition, **caractérisé en ce que** dans
- une première étape de procédé, les oléfines sont mises à réagir au moyen d'un catalyseur complexe de métal de transition soluble dans l'eau à base d'un métal et de ligands y étant reliés, en présence d'un solvant miscible à l'eau, où la pression, la température et les proportions en quantité du solvant et de la solution de catalyseur aqueuse sont contrôlées de telle manière que l'hydroformylation est effectuée dans une solution réactionnelle monophasique homogène, et dans
- une deuxième étape de procédé, la solution réactionnelle homogène obtenue à partir de la première étape de réaction est convertie en une solution de procédé biphasique par la diminution de la température et/ou la réduction de la pression, et au moins une partie de la phase organique est séparée de la phase aqueuse.

2. Procédé selon la revendication 1, dans lequel la température et la pression sont maintenues constantes pendant la conversion et le caractère monophasique de la solution réactionnelle est contrôlé par le biais des rapports massiques de solvant sur la solution de catalyseur aqueuse.

3. Procédé selon l'une des revendications précédentes, dans lequel la première étape réactionnelle est effectuée à une pression égale ou supérieure à 0,5 MPA et inférieure ou égale à 10 MPa et à une température égale ou supérieure à 70 °C et inférieure ou égale à 150 °C.

4. Procédé selon l'une des revendications précédentes, dans lequel les oléfines présentent au moins deux doubles liaisons non conjuguées.

5. Procédé selon l'une des revendications précédentes, dans lequel les oléfines présentent au moins un cycle aliphatique.

6. Procédé selon l'une des revendications précédentes, dans lequel le rapport molaire de l'eau dans la solution de catalyseur aqueuse engagée sur le métal catalyseur, exprimé sous la forme des moles d'eau divisées par les moles de métal catalyseur, est égal ou supérieur à 5000 et inférieur ou égal à 60000.

7. Procédé selon l'une des revendications précédentes, dans lequel le métal du catalyseur complexe de métal de transition soluble dans l'eau est du rhodium et les ligands comprennent des di phosphines ou des tri arylphosphines solubles dans l'eau.

8. Procédé selon l'une des revendications précédentes, dans lequel le rapport massique de la solution de catalyseur sur le solvant, exprimé sous la forme de la masse de solution de catalyseur divisée par la masse de solvant, est égal ou supérieur à 0,25 et inférieur ou égal à 4.

9. Procédé selon l'une des revendications précédentes, dans lequel le solvant miscible avec l'eau à 20 °C présente une solubilité dans l'eau égale ou supérieure à 20 g/l.

10. Procédé selon l'une des revendications précédentes, dans lequel le solvant est choisi dans le groupe constitué d'alcools linéaires ou ramifiés en C2 à C5 ou de mélanges d'au moins deux alcools de ce groupe.

11. Procédé selon l'une des revendications précédentes, dans lequel le solvant est l'isopropanol.

12. Procédé selon l'une des revendications précédentes, dans lequel au moins un ligand du catalyseur complexe de métal de transition soluble dans l'eau comprend un sel de sodium de l'acide triphényl phosphin-3,3',3"-trisulfonique.

13. Procédé selon l'une des revendications précédentes, dans lequel le catalyseur complexe de métal de transition comprend des ligands tri arylphosphines et un métal catalyseur, où le rapport de l'engagement molaire des ligands tri arylphosphines sur le métal catalyseur, exprimé sous forme de moles de ligands de tri arylphosphine divisées par les moles de métal catalyseur, est égal ou supérieur à 3 et inférieur ou égal à 15.

14. Procédé selon l'une des revendications précédentes, dans lequel le rapport molaire du métal catalyseur sur les oléfines, exprimé sous la forme des moles de métal catalyseur sur les moles d'oléfines, est égal ou supérieur à 0,05 % et inférieur ou égal à 0,75 %.

15. Procédé selon l'une des revendications précédentes, dans lequel la valeur du pH de la solution de catalyseur aqueuse est égale ou supérieure à pH 4 et inférieure ou égale à pH 10.
